# EUROPÄISCHE PATENTSCHRIFT

(11) **EP 3 686 179 B1**
(45) Veröffentlichungstag und Bekanntmachung des Hinweises auf die Patenterteilung: **08.09.2021**
(21) Anmeldenummer: 19153767.9
(22) Anmeldetag: 25.01.2019
(51) Int. Cl.: C07C 29/152, C07C 31/04

(54) **VERFAHREN ZUM BETRIEB EINER ANLAGE FÜR DIE SYNTHESE VON METHANOL**
METHOD FOR OPERATING A PLANT FOR THE SYNTHESIS OF METHANOL
PROCÉDÉ DE FONCTIONNEMENT D'UNE INSTALLATION POUR LA SYNTHÈSE DU MÉTHANOL

(43) Veröffentlichungstag der Anmeldung: 29.07.2020
(73) Patentinhaber: thyssenkrupp Industrial Solutions AG, 45143 Essen (DE); GasConTec GmbH, 61348 Bad Homburg v. d. Höhe (DE)
(72) Erfinder: SCHULZ, Alexander, 60439 Frankfurt (DE)
(74) Vertreter: Patentanwälte Bauer Vorberg Kayser

(56) Entgegenhaltungen:
- EP-A2- 0 123 534
- EP-B1- 3 205 622
- WO-A1-2013/158343

## Beschreibung

Die Erfindung betrifft ein Verfahren zum Betrieb einer Anlage für die Synthese von Methanol gemäß dem Oberbegriff von Anspruch 1 sowie eine Anlage zur Synthese einer wasserstoffhaltigen Verbindung gemäß dem Oberbegriff von Anspruch 15.

Die Herstellung von Methanol findet regelmäßig in mehreren, prozesstechnisch hintereinander geschalteten Reaktorstufen einer Anlage für die Synthese von Methanol statt, welchen Reaktorstufen ein Synthesegasstrom mit Wasserstoff und Kohlenstoffoxiden zugeführt wird und in denen die exotherme Reaktion zur Herstellung von Methanol abläuft.

Eine solche Anlage zur Herstellung von Methanol ist aus der EP 0 123 534 A2 und der EP 3 205 622 B1 bekannt, von welcher die vorliegende Erfindung als nächstkommend ausgeht. Insbesondere aus diesem Stand der Technik ist es bekannt, zum Ermöglichen einer wirtschaftlichen Reaktion der Methanolsynthese diese Reaktion in einem erhöhten Druckbereich in den Reaktorstufen stattfinden zu lassen. Dieser erhöhte Druckbereich kann dadurch erreicht werden, dass der Synthesegasstrom durch einen Kompressor - dem Synthesegaskompressor - eine Druckerhöhung erfährt. Da das Synthesegas in einem einzigen Durchlauf der Reaktorstufen keine vollständige Umwandlung in Methanol erfährt und das den Reaktorstufen zugeführte Gas auch nicht vollständig aus Wasserstoff und Kohlenstoffoxiden besteht, verbleibt neben dem entstandenen Methanol auch ein Restgas, welches neben unreagiertem Wasserstoff und Kohlenstoffoxiden sowie Methan auch die inerten Bestandteile Stickstoff und Edelgase enthalten kann. Daher ist vorgesehen, nach einem Abtrennen des Rohmethanols einerseits das Restgas grundsätzlich wieder den Reaktorstufen für die Methanolsynthese zuzuführen und es auf diese Weise zu recyclen und andererseits einen Teil des Gases aus diesem Synthesekreislauf als Purgegas zu entfernen, um eine Anreicherung insbesondere der inerten Bestandteile im Kreislauf zu verhindern.

Damit bei dem Entfernen des Purgegases der Verlust an an sich nutzbaren Bestandteilen des Restgases - hier vor allem der Wasserstoff - begrenzt werden kann, wird ein wasserstoffhaltiges Gas aus dem Restgas abgetrennt bevor der verbliebene Teil des Restgases als Purgegas entfernt wird und noch als Brenngas verwendet werden kann. Diese Rückgewinnung von Wasserstoff ist auch deshalb wichtig, weil auf diese Weise die Stöchiometrie des Gases in den Reaktoren für die Methanolsynthese eingestellt werden kann.

Eine für Ausfälle anfällige Komponente einer solchen Anlage für die Synthese von Methanol ist der Kompressor für das Synthesegas. Wenn ein solcher Ausfall eintritt, kann die Anlage in der Regel nicht mehr für die Methanolsynthese betrieben werden. Das liegt nicht nur daran, dass das direkt von dem Synthesegaskompressor zu komprimierende Synthesegas durch den Ausfall nicht mehr den erforderlichen Druck für die Methanolsynthese aufweist. Sondern es liegt auch daran, dass das wasserstoffhaltige Gas aus der Wasserstoffrückgewinnung nicht mehr den erforderlichen Druck für eine Zuführung aufweist. Ohne das Gas aus der Wasserstoffrückgewinnung ist aber auch die im Normalbetrieb angestrebte Stöchiometriezahl nicht zu erreichen.

Ausgehend von diesem Stand der Technik besteht die Aufgabe der Erfindung daher darin, das aus dem Stand der Technik bekannte Verfahren zum Betrieb einer Anlage für die Synthese von Methanol und die aus dem Stand der Technik bekannte Anlage für die Synthese von Methanol dahingehend zu verbessern und weiterzuentwickeln, dass ein Weiterbetrieb auch bei einem Ausfall des Synthesegaskompressors möglich ist.

Bezogen auf ein Verfahren zum Betrieb einer Anlage für die Synthese von Methanol gemäß dem Oberbegriff von Anspruch 1 wird diese Aufgabe durch die Merkmale des kennzeichnenden Teils von Anspruch 1 gelöst. Bezogen auf eine Anlage zur Synthese von Methanol gemäß dem Oberbegriff von Anspruch 15 wird diese Aufgabe durch die Merkmale des kennzeichnenden Teils von Anspruch 15 gelöst.

Der Erfindung liegt die Erkenntnis zugrunde, dass eine Anlage für die Synthese von Methanol auch nach einem Ausfall des Synthesegaskompressors im Grunde dadurch weiter betrieben werden kann, dass der Strom mit dem rückgewonnenen Wasserstoff zur Kompensation des Ausfalls des Synthesegaskompressors angepasst wird. Zwar ist auch durch eine solche Anpassung mit einer Verringerung des Durchsatzes der Anlage zu rechnen, doch fällt diese Verringerung milder aus. Die Anpassung des Stroms mit dem rückgewonnenen Wasserstoff kann insbesondere derart erfolgen, dass im Ergebnis der Wasserstoff in dem Strom mit dem rückgewonnenen Wasserstoff mit einem höheren Druck den Reaktorstufen zugeführt wird als ohne diese Anpassung. Die Anpassung kann speziell dadurch erfolgen, dass die Zuführung des Gases zur Wasserstoffrückgewinnung und alternativ oder zusätzlich die Zuführung des rückgewonnenen Wasserstoffes durch Umschalten in ihrer Führung und damit in ihrem Verlauf verändert wird. Einerseits kann also die Zuführung so geändert werden, dass von vornherein Gas mit einem höheren Druck der Wasserstoffrückgewinnung zugeführt wird. Andererseits kann der rückgewonnene Wasserstoff auch so den Reaktorstufen zugeführt werden, dass er nach der Änderung eine Druckerhöhung erfährt. Auf diese Weise wird also der negative Effekt des Ausfalls des Synthesegaskompressors zumindest teilweise gemildert und ein Weiterbetrieb der Anlage zur Synthese von Methanol ermöglicht.

Das vorschlagsgemäße Verfahren dient dem Betrieb einer Anlage für die Synthese von Methanol. Bei dem vorschlagsgemäßen Verfahren wird ein Synthesegasstrom mit Wasserstoff und Kohlenstoffoxiden einem Synthesegaskompressor der Anlage zur Druckerhöhung des Synthesegasstroms zugeführt. Der Synthesegasstrom weist also Wasserstoff, Kohlenstoffmonoxid und Kohlenstoffdioxid auf und kann daneben noch weitere Bestandteile wie insbesondere Stickstoff und Edelgase aufweisen. Ebenso wird bei dem vorschlagsgemäßen Verfahren der druckerhöhte Synthesegasstrom einer Methanol-Reaktoranordnung der Anlage zur teilweisen Umwandlung in Methanol zugeführt. Das Merkmal der teilweisen Umwandlung in Methanol begründet sich darin, dass ein nicht umgesetzter Rest an Edukten aus der Methanol-Reaktoranordnung austritt und daher die Umwandlung nicht vollständig abläuft.

In dem vorschlagsgemäßen Verfahren weist die Anlage eine Wasserstoffrückgewinnungsanordnung auf, welche aus einem von der Methanol-Reaktoranordnung zugeführten Rückgewinnungsstrom einen H-Recyclestrom mit Wasserstoff gewinnt. In dem vorschlagsgemäßen Verfahren wird dieser Wasserstoff zumindest teilweise in Methanol umgewandelt. Diese teilweise Umwandlung in Methanol erfolgt insbesondere dadurch, dass zumindest ein Teil des Wasserstoffs des H-Recyclestroms direkt oder indirekt wieder der Methanol-Reaktoranordnung zugeführt wird und dort zu Methanol reagiert. Wie untenstehend noch beschrieben wird, kann diese Zuführung des Wasserstoffs zu der Methanol-Reaktoranordnung indirekt derart erfolgen, dass der Wasserstoff als Teil einer Reihe weiterer Ströme der Methanol-Reaktoranordnung zugeführt wird.

Das vorschlagsgemäße Verfahren ist dadurch gekennzeichnet, dass auf einen Ausfall des Synthesegaskompressors der Synthesegasstrom weiterhin der Methanol-Reaktoranordnung zur teilweisen Umwandlung in Methanol zugeführt wird.

Das vorschlagsgemäße Verfahren ist weiter dadurch gekennzeichnet, dass nach dem Ausfall des Synthesegaskompressors eine Leitungsanordnung der Anlage derart umgeschaltet ist, dass der H-Recyclestrom zur Kompensierung eines Druckverlusts in der Methanol-Reaktoranordnung angepasst wird. Diese Anpassung des H-Recyclestroms kann seine Zusammensetzung, seinen Druck und alternativ oder zusätzlich seinen Verlauf betreffen. Die Umschaltung der Leitungsanordnung der Anlage bedeutet, dass ein im Grunde beliebiger Strom der Anlage nach dem Umschalten anders geführt wird als vor dem Umschalten, wobei dieses Umschalten direkt oder indirekt die Anpassung des H-Recyclestroms bewirkt. Insbesondere kann das Umschalten der Leitungsanordnung bedeuten, dass vor dem Umschalten der fragliche Strom durch eine erste Teilleitung der Leitungsanordnung geführt wird und nach dem Umschalten dieser Strom durch eine zweite Teilleitung der Leitungsanordnung geführt wird. Die zweite Teilleitung ist dann von der ersten Teilleitung verschieden und weist beispielsweise einen anderen Verlauf auf.

Der eigentliche Vorgang des Umschaltens kann eine im Prinzip beliebige Zeit nach dem erfolgten Ausfall des Synthesegaskompressors und im Grunde auch vor einem erwarteten Ausfall des Synthesegaskompressors erfolgen. Ein bewusstes Abschalten des Synthesegaskompressors steht dabei einem unbeabsichtigten Ausfall des Synthesegaskompressors gleich. Mithin ist es also unerheblich, weswegen der Synthesegaskompressor ausgefallen ist. Das Umschalten kann auch mehr als eine Leitungsanordnung und damit mehrere Leitungsanordnungen betreffen. Insbesondere können durch das Umschalten auch gleich mehrere Ströme in ihrer Führung verändert werden.

Grundsätzlich kann die teilweise Umwandlung des druckerhöhten Synthesegasstroms in der Methanol-Reaktoranordnung in einem weiten Druckbereich erfolgen. Besonders vorteilhaft ist es aber, den Synthesegasstrom bei einem möglichst hohen Druck bereitzustellen. Daher ist es bevorzugt, dass auf den Ausfall des Synthesegaskompressors und nach dem Umschalten der Leitungsanordnung der Synthesegasstrom der Methanol-Reaktoranordnung mit einem Druck von mindestens 40 bar und insbesondere mit einem Druck von mindestens 50 bar zugeführt wird. Auf diese Weise ist gewährleistet, dass die Methanolsynthese auch nach dem Ausfall des Synthesegaskompressors in einem Druckbereich stattfindet, der trotz eines reduzierten Durchsatzes einen wirtschaftlichen Betrieb der Anlage erlaubt.

Es kann eine Anlage zur Synthese von Methanol neben dem Kompressor für das insbesondere frische Synthesegas mindestens einen weiteren Kompressor - auch als Recyclekompressor bezeichnet - aufweisen, welcher unreagiertes Restgas aus den Methanol-Reaktoren für eine Rückführung zu den Methanol-Reaktoren komprimiert. Entsprechend ist eine bevorzugte Ausführungsform des Verfahrens dadurch gekennzeichnet, dass aus der Methanol-Reaktoranordnung ein Restgasstrom mit unreagiertem Restgas gewonnen wird und dass die Anlage einen Recyclekompressor zur Druckerhöhung des Restgasstroms und zur Zuführung des druckerhöhten Restgasstroms zur Methanol-Reaktoranordnung zur teilweisen Umwandlung in Methanol aufweist. Vorzugsweise umfasst die Methanol-Reaktoranordnung eine Methanol-Trennvorrichtung zum Gewinnen des unreagierten Restgases und eines Rohmethanolstroms. Grundsätzlich kann die Methanol-Trennvorrichtung auf beliebige Art und Weise funktionieren. Insbesondere kann es sein, dass die Methanol-Trennvorrichtung eine Kondensationsvorrichtung zum Gewinnen des unreagierten Restgases und des Rohmethanolstroms durch Kondensation umfasst.

Prinzipiell kann es sein, dass die Methanol-Reaktoranordnung nur eine einzige Methanol-Reaktorstufe umfasst. Eine weitere bevorzugte Ausführungsform des Verfahrens ist dadurch gekennzeichnet, dass die Methanol-Reaktoranordnung eine Vielzahl von prozesstechnisch hintereinander geschalteten Reaktorstufen zur Methanolsynthese aufweist. Jede einzelne Reaktorstufe kann dabei einen oder mehrere Reaktoren aufweisen. Die Reaktoren einer Reaktorstufe können dabei insbesondere untereinander prozesstechnisch parallel angeordnet sein.

Dass die Reaktorstufen prozesstechnisch hintereinander geschaltet sind bedeutet, dass Restgas aus einer Reaktorstufe - sofern es nicht die letzte Reaktorstufe in der Reihe der Reaktorstufen ist - direkt oder indirekt der jeweils danach geschalteten Reaktorstufe zugeführt wird. Grundsätzlich kann der obige Recyclekompressor bezüglich der Vielzahl von Reaktorstufen beliebig angeordnet sein. Eine Variante ist, dass der Recyclekompressor prozesstechnisch zwischen zwei Reaktorstufen angeordnet ist. Das bedeutet, dass dem Recyclekompressor zumindest ein Teil eines Gases aus einer Reaktorstufe zugeführt wird und der druckerhöhte Restgasstrom dann der dieser Reaktorstufe nachgelagerten Reaktorstufe zugeführt wird.

Gemäß einer bevorzugten Ausführungsform des Verfahrens ist vorgesehen, dass der druckerhöhte Synthesegasstrom einer ersten Reaktorstufe der Vielzahl von Reaktorstufen zugeführt wird. Weiter ist bevorzugt, dass der Restgasstrom aus einem prozesstechnisch der ersten Reaktorstufe nachgelagerten Reaktorstufe gewonnen wird. Mit anderen Worten entstammt dann der dem Recyclekompressor zugeführte Restgasstrom nicht der ersten Reaktorstufe - also der Reaktorstufe, welcher der Synthesegasstrom unmittelbar zugeführt wird - sondern einer nachgelagerten Reaktorstufe. Weiter kann es sein, dass der Recyclekompressor den druckerhöhten Restgasstrom der ersten Reaktorstufe zuführt. Grundsätzlich kann der erhöhte Restgasstrom aber auch einer anderen Reaktorstufe der Vielzahl von Reaktorstufen zugeführt werden.

Grundsätzlich kann der H-Recyclestrom beliebig geführt werden, solange mindestens ein Teil seines Wasserstoffs in Methanol umgewandelt wird. Gemäß einer weiteren bevorzugten Ausführungsform des Verfahrens ist diesbezüglich bevorzugt, dass nach dem Umschalten der Leitungsanordnung der H-Recyclestrom dem unreagierten Restgas zugeführt wird. Mit anderen Worten wird der Wasserstoff des H-Recyclestroms nach der Zuführung gemeinsam mit zumindest einem Teil des unreagierten Restgases behandelt. Hier ist es weiter bevorzugt, dass nach dem Umschalten der Leitungsanordnung der H-Recyclestrom dem Recyclekompressor zur Druckerhöhung gemeinsam mit dem Restgasstrom zugeführt wird. Durch den Recyclekompressor kann der H-Recyclestrom dann eine Druckerhöhung erfahren, welche die nach dem Ausfall des Synthesegaskompressors fehlende Druckerhöhung durch diesen kompensiert.

Es kann also das Umschalten den Verlauf des H-Recyclestroms betreffen. Das Umschalten kann aber auch den Verlauf anderer Ströme betreffen. So ist eine bevorzugte Ausführungsform des Verfahrens dadurch gekennzeichnet, dass durch das Umschalten der Leitungsanordnung der Verlauf des Rückgewinnungsstroms geändert wird. Vorzugsweise wird nämlich vor dem Umschalten der Leitungsanordnung der Rückgewinnungsstrom aus dem unreagierten Restgas abgezweigt. Dieses Abzweigen kann insbesondere dem Recyclekompressor prozesstechnisch vorgelagert erfolgen. Der abgezweigte Rückgewinnungsstrom hat in so einem Fall die Druckerhöhung durch den Recyclekompressor nicht erfahren, weswegen dann eine Druckerhöhung des H-Recyclestroms durch den Synthesegaskompressor sinnvoll ist.

Umgekehrt ist es dann bei einem Ausfall des Synthesegaskompressors vorteilhaft, bereits für den Rückgewinnungsstrom eine Druckerhöhung zu erreichen, wodurch mittelbar auch der H-Recyclestrom einen höheren Druck aufweist. Eine weitere bevorzugte Ausführungsform des Verfahrens ist daher dadurch gekennzeichnet, dass durch das Umschalten der Leitungsanordnung der Rückgewinnungsstrom mit einem erhöhten Druck der Wasserstoffrückgewinnungsanordnung zugeführt wird. Vorzugsweise wird durch das Umschalten der Leitungsanordnung der Rückgewinnungsstrom mit einem höheren Druck als vor dem Umschalten der Leitungsanordnung zugeführt, und zwar insbesondere der Wasserstoffrückgewinnungsanordnung zugeführt. Eine bevorzugte Möglichkeit zur Erhöhung des Drucks des Rückgewinnungsstroms besteht darin, diesen zuvor durch den Recyclekompressor komprimieren zu lassen. Entsprechend ist es bevorzugt, dass nach dem Umschalten der Leitungsanordnung der Rückgewinnungsstrom dem Recyclekompressor prozesstechnisch nachgelagert aus dem Restgasstrom abgezweigt wird.

Gemäß einer bevorzugten Ausführungsform des Verfahrens wird durch das Umschalten der Leitungsanordnung der Verlauf des H-Recyclestroms geändert. Vorzugsweise wird vor dem Umschalten der Leitungsanordnung der H-Recyclestrom dem Synthesegasstrom dem Synthesegaskompressor prozesstechnisch vorgelagert zugeführt. Anders ausgedrückt wird vor dem Umschalten der Leitungsanordnung der H-Recyclestrom dem Synthesegasstrom zugeführt und diese Zuführung zum Synthesegasstrom erfolgt dem Synthesegaskompressor prozesstechnisch vorgelagert. Auf diese Weise erfährt der H-Recyclestrom gemeinsam mit dem Synthesegasstrom eine Druckerhöhung durch den Synthesegaskompressor und der Rückgewinnungsstrom kann mit vergleichsweise geringem Druck der Wasserstoffrückgewinnungsanordnung zugeführt werden.

Gemäß einer weiteren bevorzugten Ausführungsform des Verfahrens ist es bevorzugt, dass durch das Umschalten der Leitungsanordnung der Wasserstoff des H-Recyclestroms der Methanol-Reaktoranordnung mit einem erhöhten Druck zur teilweisen Umwandlung in Methanol zugeführt wird. Der Druck des Wasserstoffs ist dann erhöht gegenüber dem Druck des Wasserstoffs ohne das Umschalten der Leitungsanordnung. Anders ausgedrückt ist es bevorzugt, dass durch das Umschalten der Leitungsanordnung der Wasserstoff des H-Recyclestroms der Methanol-Reaktoranordnung mit einem höheren Druck als vor dem Umschalten der Leitungsanordnung zugeführt wird. Insbesondere gilt dies nach Ausfall des Synthesegaskompressors. Vorzugsweise wird der H-Recyclestrom, insbesondere insgesamt, durch das Umschalten der Leitungsanordnung der Methanol-Reaktoranordnung mit einem höheren Druck als vor dem Umschalten der Leitungsanordnung zugeführt.

Ein solcher erhöhter Druck kann einerseits dadurch bewirkt werden, dass der H-Recyclestrom eine direkte Druckerhöhung oder eine indirekte Druckerhöhung erfährt. Eine indirekte Druckerhöhung kann - wie bereits festgestellt - etwa durch eine Druckerhöhung des Rückgewinnungsstroms erreicht werden. Es kann aber auch sein, dass der Wasserstoff auch dann der Methanol-Reaktoranordnung mit einem erhöhten Druck zugeführt wird, wenn der H-Recyclestrom selbst keine Druckerhöhung erfährt.

Eine bevorzugte Ausführungsform des Verfahrens ist dadurch gekennzeichnet, dass der Synthesegasstrom in einer Synthesegasreaktoranordnung der Anlage aus einem kohlenstoffhaltigen Energieträgerstrom gewonnen wird. Die Synthesegasreaktoranordnung kann dabei neben einem Reaktor zur Erzeugung des Synthesegases weitere Vorrichtungen aufweisen. So kann die Synthesegasreaktoranordnung eine jeweils dem Reaktor prozesstechnisch vorgelagerte Vorrichtung zur Entschwefelung des kohlenstoffhaltigen Energieträgerstroms, eine Sättigungsstufe zur Sättigung des kohlenstoffhaltigen Energieträgerstroms mit Wasser, einen Pre-Reformer zur Vorreformierung des kohlenstoffhaltigen Energieträgerstroms und/oder eine Vorrichtung zum Aufheizen des kohlenstoffhaltigen Energieträgerstroms aufweisen.

Grundsätzlich kann dieses Gewinnen des Synthesegasstroms auf beliebige Art und Weise erfolgen. Bevorzugt ist, dass zum Gewinnen des Synthesegasstroms ein sauerstoffhaltiger Strom der Synthesegasreaktoranordnung zugeführt wird. Grundsätzlich kann der sauerstoffhaltige Strom neben dem Sauerstoff noch weitere Bestandteile aufweisen. So kann es sich bei dem sauerstoffhaltigen Strom auch um Umgebungsluft handeln. Weiter kann es sein, dass vor dem Umschalten der H-Recyclestrom dem Synthesegasstrom der Synthesegasreaktoranordnung prozesstechnisch nachgelagert zugeführt wird. Bei funktionierendem Synthesegaskompressor ist es zweckmäßiger, den wiedergewonnenen Wasserstoff nicht durch die Synthesegasreaktoranordnung zu führen.

Grundsätzlich kann der Synthesegasstrom etwa durch eine Dampfreformierung des kohlenstoffhaltigen Energieträgerstroms gewonnen werden. Eine weitere bevorzugte Ausführungsform des Verfahrens ist dadurch gekennzeichnet, dass in der Synthesegasreaktoranordnung der Synthesegasstrom durch eine autotherme Reformierung aus dem kohlenstoffhaltigen Energieträgerstrom gewonnen wird. Bei einer solchen autothermen Reformierung stellt eine katalytische partielle Oxidation die für die endothermen Reformierungsreaktionen erforderliche Wärme bereit. Gegenüber einer reinen Dampfreformierung bietet die autotherme Reformierung den Vorteil, dass der Synthesegasstrom mit einem höheren Druck bereitgestellt werden kann.

Grundsätzlich kann eine solche autotherme Reformierung auch mit Umgebungsluft betrieben werden. Bevorzugt ist jedoch, dass der sauerstoffhaltige Strom aus einer Luftzerlegungsvorrichtung zum Gewinnen eines Sauerstoffstroms aus einer Umgebungsluft gewonnen wird. Die Luftzerlegungsvorrichtung kann darüber hinaus auch zum Gewinnen eines Stickstoffstroms eingerichtet sein. Insbesondere kann es dann sein, dass der sauerstoffhaltige Strom im Wesentlichen aus Sauerstoff besteht. Auf diese Weise wird der Anteil an inerten Gasen bei der Methanolsynthese verringert, sodass u.a. Kompressoren und andere Vorrichtungen der Anlage kleiner dimensioniert werden können.

Gemäß einer bevorzugten Ausführungsform des Verfahrens ist vorgesehen, dass nach dem Umschalten der Leitungsanordnung der H-Recyclestrom dem Energieträgerstrom zugeführt wird. Insbesondere kann es sein, dass nach dem Umschalten der Leitungsanordnung der H-Recyclestrom dem Energieträgerstrom der Synthesegasreaktoranordnung prozesstechnisch vorgelagert zugeführt wird. Auf diese Weise kann der etwa bei einer autothermen Reformierung erreichte Druck auch auf den Wasserstoff des H-Recyclestroms ausgedehnt werden. Ebenso kann es sein, dass die Anlage einen Energieträgerkompressor zur Druckerhöhung des Energieträgerstroms vor Zuführung zu der Synthesegasreaktoranordnung aufweist und dass nach dem Umschalten der Leitungsanordnung der H-Recyclestrom dem Energieträgerstrom dem Energieträgerkompressor vorgelagert zugeführt wird. Auf diese Weise kann durch den Energieträgerkompressor auch der Wasserstoff in dem H-Recyclestrom eine Druckerhöhung erfahren.

Neben dem H-Recyclestrom kann die Wasserstoffrückgewinnungsanordnung auch weitere Ströme ausgeben. Vorzugsweise gibt die Wasserstoffrückgewinnungsanordnung einen Purgestrom aus. Dieser kann insbesondere zur Verfeuerung abgeführt werden.

Grundsätzlich kann der H-Recyclestrom eine beliebige Zusammensetzung aufweisen, sofern er Wasserstoff enthält. Gemäß einer weiteren bevorzugten Ausführungsform des Verfahrens ist vorgesehen, dass der H-Recyclestrom einen höheren molaren Anteil an Wasserstoff als der Rückgewinnungsstrom aufweist. Mit anderen Worten ist in dem H-Recyclestrom der Wasserstoff gegenüber dem Rückgewinnungsstrom angereichert. Ebenso ist es bevorzugt, dass der H-Recyclestrom einen höheren molaren Anteil an Wasserstoff als der Purgestrom aufweist.

Grundsätzlich kann die Wasserstoffrückgewinnungsanordnung nach einem beliebigen Prinzip funktionieren, so etwa basierend auf einer Membran oder einer Kältevorrichtung. Eine bevorzugte Ausführungsform des Verfahrens ist dadurch gekennzeichnet, dass die Wasserstoffrückgewinnungsanordnung eine Druckwechsel-Adsorptionsvorrichtung (PSA) zum Gewinnen des H-Recyclestroms aus dem Rückgewinnungsstrom aufweist. Auf diese Weise kann eine hohe Rückgewinnung an Wasserstoff im H-Recyclestrom erreicht werden. Eine hohe Wasserstoffreinheit ist dabei zwar nicht erforderlich, kann aber erreicht werden. Es kann also sein, dass der H-Recyclestrom im Wesentlichen aus Wasserstoff besteht.

Die vorschlagsgemäße Anlage dient der Synthese von Methanol. Sie weist einen Synthesegaskompressor, welchem ein Synthesegasstrom mit Wasserstoff und Kohlenstoffoxiden zur Druckerhöhung des Synthesegasstroms zugeführt wird, eine Methanol-Reaktoranordnung, welcher der druckerhöhte Synthesegasstrom zur teilweisen Umwandlung in Methanol zugeführt wird und eine Wasserstoffrückgewinnungsanordnung zum Gewinnen eines H-Recyclestroms mit Wasserstoff aus einem von der Methanol-Reaktoranordnung zugeführten Rückgewinnungsstrom auf, wobei der Wasserstoff zumindest teilweise in Methanol umgewandelt wird.

Die vorschlagsgemäße Anlage ist dadurch gekennzeichnet, dass die Anlage eine Leitungsanordnung und eine Schaltanordnung aufweist, welche Schaltanordnung auf einen Ausfall des Synthesegaskompressors die Leitungsanordnung derart umschaltet, dass der H-Recyclestrom zur Kompensierung eines Druckverlusts in der Methanol-Reaktoranordnung angepasst wird.

Merkmale, Vorteile und Eigenschaften der vorschlagsgemäßen Anlage entsprechen den Merkmalen, Vorteilen und Eigenschaften des vorschlagsgemäßen Verfahrens und umgekehrt.

Weitere Einzelheiten, Merkmale, Ziele und Vorteile der vorliegenden Erfindung werden nachfolgend anhand der nur Ausführungsbeispiele wiedergebenden Zeichnung erläutert. In der Zeichnung zeigt
- Fig. 1: schematisch das Fließbild einer Anlage zur Ausführung des vorschlagsgemäßen Verfahrens gemäß einem ersten Ausführungsbeispiel,
- Fig. 2: schematisch das Fließbild einer Anlage zur Ausführung des vorschlagsgemäßen Verfahrens gemäß einem zweiten Ausführungsbeispiel,
- Fig. 3: schematisch das Fließbild einer Anlage zur Ausführung des vorschlagsgemäßen Verfahrens gemäß einem dritten Ausführungsbeispiel,
- Fig. 4: schematisch das Fließbild einer Anlage zur Ausführung des vorschlagsgemäßen Verfahrens gemäß einem vierten Ausführungsbeispiel,
- Fig. 5: schematisch das Fließbild einer Anlage zur Ausführung des vorschlagsgemäßen Verfahrens gemäß einem fünften Ausführungsbeispiel,
- Fig. 6: schematisch das Fließbild einer Anlage zur Ausführung des vorschlagsgemäßen Verfahrens gemäß einem sechsten Ausführungsbeispiel und
- Fig. 7: schematisch das Fließbild einer Anlage zur Ausführung des vorschlagsgemäßen Verfahrens gemäß einem siebten Ausführungsbeispiel.

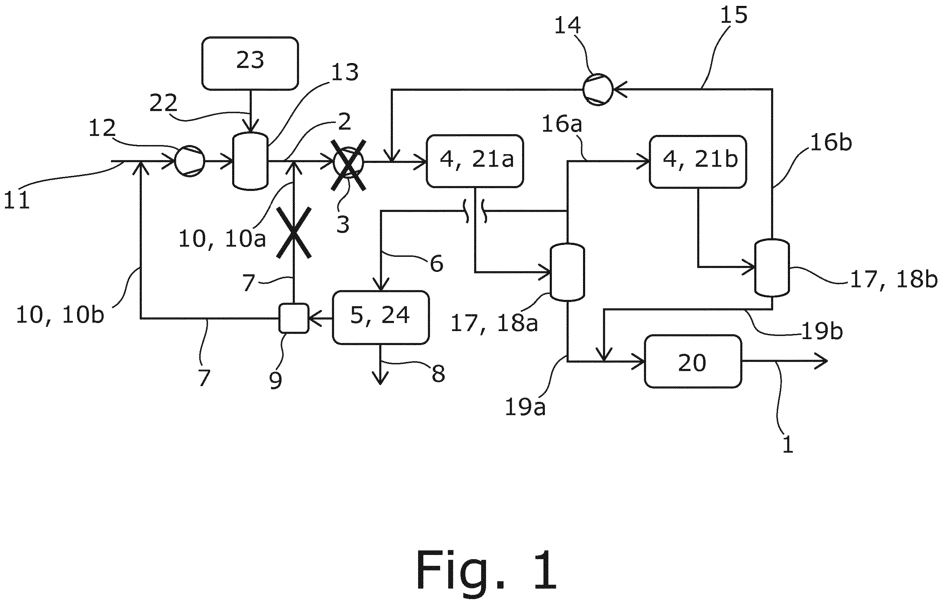

Die in der Fig. 1 gezeigte Anlage gemäß einem ersten Ausführungsbeispiel der vorschlagsgemäßen Anlage dient der Synthese von Methanol 1 und kann gemäß dem vorschlagsgemäßen Verfahren betrieben werden.

Ein im Wesentlichen aus Wasserstoff, Kohlenstoffmonoxid und Kohlenstoffdioxid bestehender Synthesegasstrom 2 wird einem Synthesegaskompressor 3 der Anlage zugeführt, welcher den Synthesegasstrom 2 auf einen gewünschten Druck für die Methanolsynthese komprimiert und anschließend einer Methanol-Reaktoranordnung 4 zugeführt, in welcher eine Methanolsynthese stattfindet und zumindest ein Teil des Synthesegasstroms 2 in Methanol 1 umgewandelt wird.

Die Anlage weist eine Druckwechsel-Adsorptionsanlage 24 - welche auch als PSA bezeichnet werden kann - ausgebildete Wasserstoffrückgewinnungsanordnung 5 auf, welche aus einem Rückgewinnungsstrom 6 von der Methanol-Reaktoranordnung 4 einen H-Recyclestrom 7 gewinnt, welcher im Wesentlichen aus Wasserstoff besteht. Ebenso wird das verbleibende Gas von der Wasserstoffrückgewinnungsanordnung 5 als Purgestrom 8 ausgegeben und anschließend in einer - hier nicht dargestellten - befeuerten Heizvorrichtung der Anlage verfeuert.

Im Normalbetrieb, also mit funktionierendem Synthesegaskompressor 3, wird der H-Recyclestrom 7 dem Synthesegasstrom 2 zugeführt, und zwar dem Synthesegaskompressor 3 vorgelagert. Der Synthesegaskompressor 3 komprimiert auf diese Weise auch den Wasserstoff in dem H-Recyclestrom 7 und führt ihm der Methanol-Reaktoranordnung 4 zu.

Der Synthesegasstrom 2 wird aus einem durch Erdgas gebildeten und damit kohlenstoffhaltigen Energieträgerstrom 11 gewonnen, welcher zunächst einem Energieträgerkompressor 12 und nach der entsprechenden Druckerhöhung einer Synthesegasreaktoranordnung 13 zugeführt. In der Synthesegasreaktoranordnung 13 findet eine autotherme Reformierung zum Gewinnen des Synthesegasstroms 2 statt. Für die autotherme Reformierung wird ein sauerstoffhaltiger Strom 22 zugeführt, welcher hier aus einer Luftzerlegungsvorrichtung 23 gewonnen wurde und im Wesentlichen aus Sauerstoff besteht. Die Luftzerlegungsvorrichtung 23 ist dabei zum Gewinnen eines Sauerstoffstroms - hier also des sauerstoffhaltigen Stroms 22 - aus der Umgebungsluft eingerichtet.

Nach einem Ausfall des Synthesegaskompressors 3 wird die Anlage und insbesondere die Methanol-Reaktoranordnung 4 weiter betrieben. Zur Kompensation des Ausfalls des Synthesegaskompressors 3 wird durch eine Schaltanordnung 9 der Anlage die Leitungsanordnung 10, welche den H-Recyclestrom 7 führt, umgeschaltet, sodass der H-Recyclestrom 7 nach dem Ausfall des Synthesegaskompressors 3 und nach dem Umschalten dem Energieträgerstrom 11 zugeführt wird. Speziell erfolgt diese Zuführung sowohl der Synthesegasreaktoranordnung 13 als auch dem Energieträgerkompressor 12 prozesstechnisch vorgelagert. In der Fig. 1 ist sowohl die vor dem Umschalten zum Führen des H-Recyclestroms 7 verwendete Teilleitung 10a der Leitungsanordnung 10 als auch die nach dem Ausfall des Synthesegaskompressors 3 und nach dem Umschalten zum Führen des H-Recyclestroms 7 verwendete Teilleitung 10b der Leitungsanordnung 10 dargestellt.

Durch den Energieträgerkompressor 12 erfährt der Wasserstoff in dem H-Recyclestrom 7 nach dem Umschalten eine Druckerhöhung gegenüber dem Zustand vor dem Umschalten, welche Druckerhöhung den Druckverlust in der Methanol-Reaktoranordnung 4 aus dem Ausfall des Synthesegaskompressors 3 zumindest teils kompensiert.

Wie in der Fig. 1 zu erkennen ist, weist die Anlage des ersten Ausführungsbeispiels auch einen Recyclekompressor 14 auf, welcher einen Restgasstrom 15 komprimiert. Der Restgasstrom 15 weist unreagiertes Restgas 16b auf, welches seinerseits im Wesentlichen diejenigen Bestandteile des Synthesegases aufweist, welche in der Methanol-Reaktoranordnung 4 nicht in Methanol 1 umgewandelt wurden. Der somit druckerhöhte Restgasstrom 15 wird erneut der Methanol-Reaktoranordnung 4 zugeführt.

Das unreagierte Restgas 16a, b wird aus einer Methanol-Trennvorrichtung 17 der Methanol-Reaktoranordnung 4 gewonnen, welche hier zwei Kondensationsvorrichtungen 18a, b umfasst. Durch Kondensation wird in diesen jeweils das unreagierte Restgas 16a, b einerseits und ein jeweiliger Rohmethanolstrom 19a, b andererseits gewonnen. Die Rohmethanolströme 19a, b werden dann einer Destillation 20 der Anlage zugeführt, sodass das Methanol 1 aus den Rohmethanolströmen 19a, b gewonnen werden kann.

Bei der Anlage des Ausführungsbeispiels der Fig. 1 weist die Methanol-Reaktoranordnung 4 zwei prozesstechnisch hintereinander geschaltete Reaktorstufen 21a, b zur Methanolsynthese auf. Bei diesem Ausführungsbeispiel weist die erste Reaktorstufe 21a zwei zueinander parallel angeordnete isotherme Reaktoren und die zweite Reaktorstufe 21b einen einzelnen isothermen Reaktor auf. Jeder der beiden Kondensationsvorrichtungen 18a, b wird dabei der Produktstrom aus jeweils einer Reaktorstufe 21a, b zugeführt. Dabei wird diejenige Reaktorstufe 21a, welcher der Synthesegasstrom 1 direkt zugeführt wird, als erste Reaktorstufe 21a bezeichnet. Die Reaktorstufe 21b ist dieser dann in dem Sinne prozesstechnisch nachgelagert, dass ihr das unreagierte Restgas 16a aus der ersten Reaktorstufe 21a zur Umwandlung in Methanol 1 zugeführt wird.

Bei diesem Ausführungsbeispiel der Fig. 1 wird der Rückgewinnungsstrom 6 aus dem unreagierten Restgas 16a der ersten Reaktorstufe 21a abgezweigt. Hingegen wird der dem Recyclekompressor 14 zugeführte Restgasstrom 15 nicht aus diesem unreagierten Restgas 16a der ersten Reaktorstufe 21a gewonnen, sondern aus dem unreagierten Restgas 16b der der ersten Reaktorstufe 21a prozesstechnisch nachgelagerten Reaktorstufe 21b. Der durch den Recyclekompressor 14 komprimierte Restgasstrom 15 wird dann wiederum der ersten Reaktorstufe 21a zugeführt.

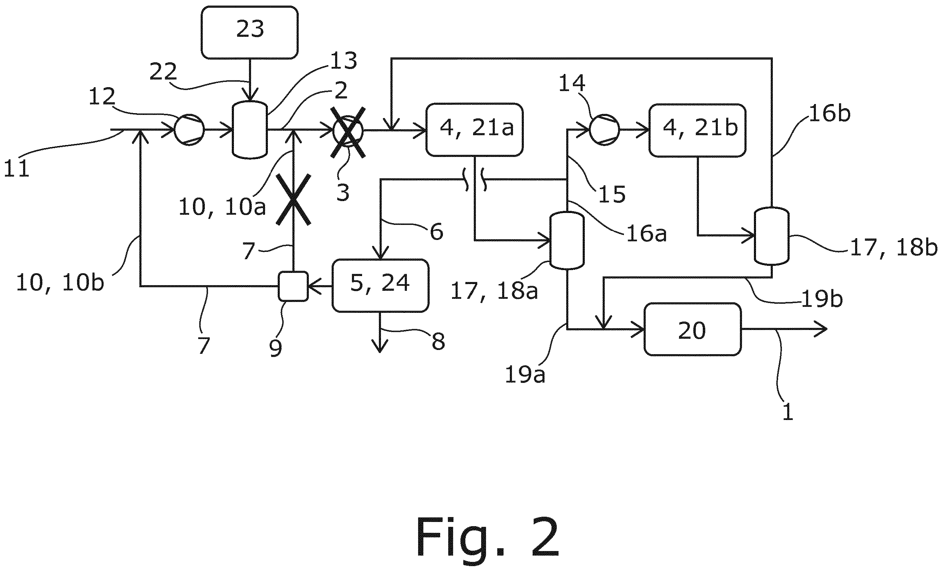

Das zweite Ausführungsbeispiel der vorschlagsgemäßen Anlage, dargestellt in der Fig. 2, unterscheidet sich von dem Ausführungsbeispiel der Fig. 1 lediglich darin, dass der Recyclekompressor 14 prozesstechnisch zwischen der ersten Reaktorstufe 21a und der ihr nachgelagerten Reaktorstufe 21b angeordnet ist. Folglich wird der dem Recyclekompressor 14 zugeführte Restgasstrom 15 aus dem unreagierten Restgas 16a der ersten Reaktorstufe 21a gewonnen. Der durch den Recyclekompressor 14 komprimierte Restgasstrom 15 wird der der ersten Reaktorstufe 21a nachgelagerten Reaktorstufe 21b zugeführt. Das unreagierte Restgas 16b aus dieser Reaktorstufe 21b wird ohne weitere Komprimierung zurück zur ersten Reaktorstufe 21a geführt. Der Rückgewinnungsstrom 6 wird - ebenso wie bei dem ersten Ausführungsbeispiel - aus dem unreagierten Restgas 16a der ersten Reaktorstufe 21a gewonnen, wobei das Abzweigen des Rückgewinnungsstroms 6 dem Recyclekompressor 14 prozesstechnisch vorgelagert erfolgt.

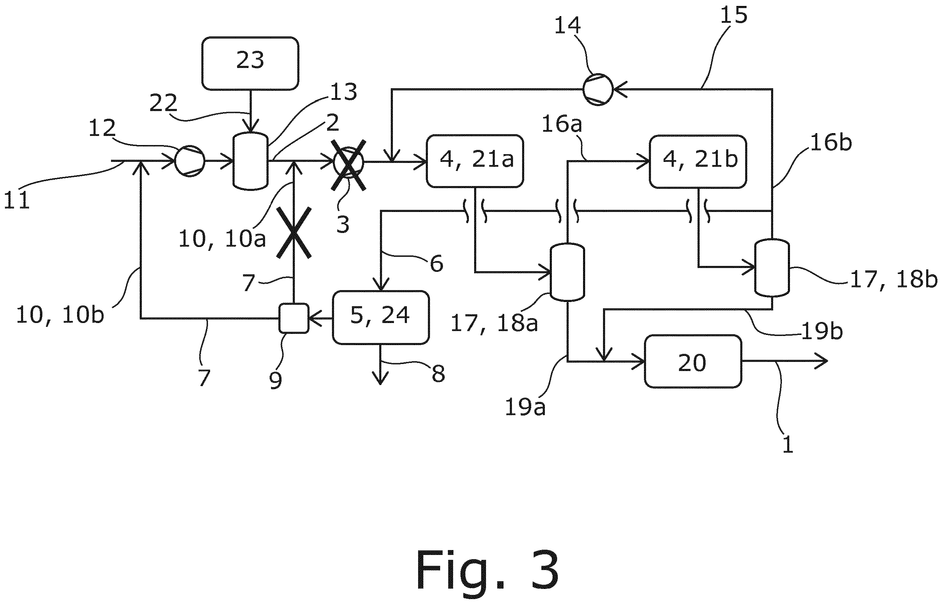

Das dritte Ausführungsbeispiel der vorschlagsgemäßen Anlage der Fig. 3 entspricht dem ersten Ausführungsbeispiel der Fig. 1 mit dem Unterschied, dass der Restgasstrom 6 aus dem unreagierten Restgas 16b der der ersten Reaktorstufe 21a nachgelagerten Reaktorstufe 21b gewonnen wird. Die Abzweigung des Restgasstroms 6 erfolgt dem Recyclekompressor 14 prozesstechnisch vorgelagert.

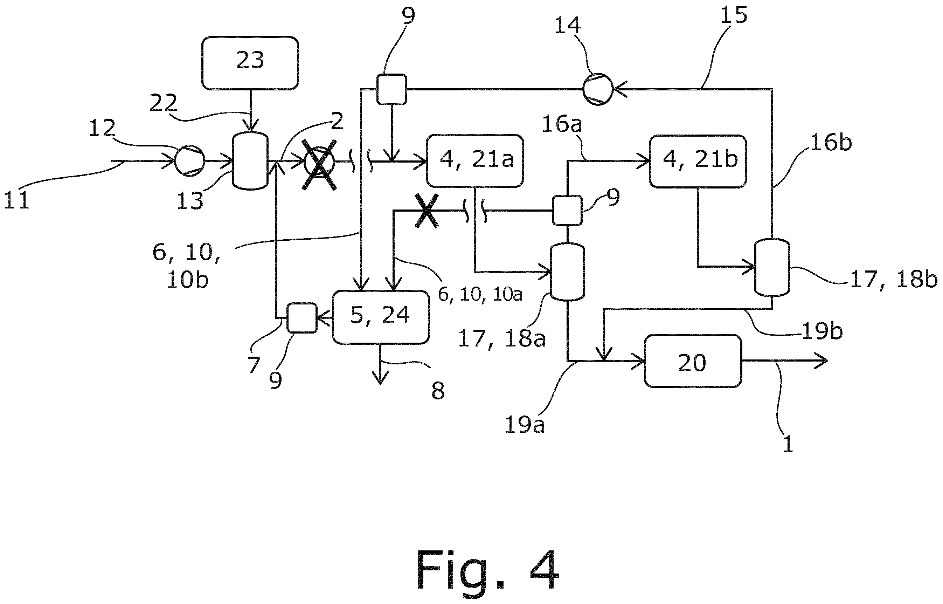

Das vierte Ausführungsbeispiel der vorschlagsgemäßen Anlage der Fig. 4 entspricht dem ersten Ausführungsbeispiel der Fig. 1 in dem Zustand mit Synthesegaskompressor 3 im Betrieb. Auf einen Ausfall des Synthesegaskompressors 3 erfolgt hier jedoch im Gegensatz zu den Ausführungsbeispielen der Fig. 1 bis 3 durch das Umschalten keine Änderung des Verlaufs des H-Recyclestroms 7, sondern vielmehr eine Änderung des Verlaufs des Rückgewinnungsstroms 6. Durch das Umschalten der Leitungsanordnung 10 wird der Rückgewinnungsstrom 6 nicht mehr durch die Teilleitung 10a der Leitungsanordnung 10 und damit nicht mehr aus dem unreagierten Restgas 16a der ersten Reaktorstufe 21a abgezweigt, sondern vielmehr aus dem von dem Recyclekompressor 14 druckerhöhten Restgasstrom 15 und somit durch die Teilleitung 10b der Leitungsanordnung 10. Dadurch, dass der Rückgewinnungsstrom 6 nach dem Umschalten eine Druckerhöhung durch den Recyclekompressor 14 erfährt, wird also der Rückgewinnungsstrom 6 mit einem höheren Druck als vor dem Umschalten der Wasserstoffrückgewinnungsanordnung 5 zugeführt. Daraus folgt aber im Ergebnis auch eine Druckerhöhung des H-Recyclestroms 7, auch wenn die Führung des H-Recyclestroms 7 an sich unverändert bleibt.

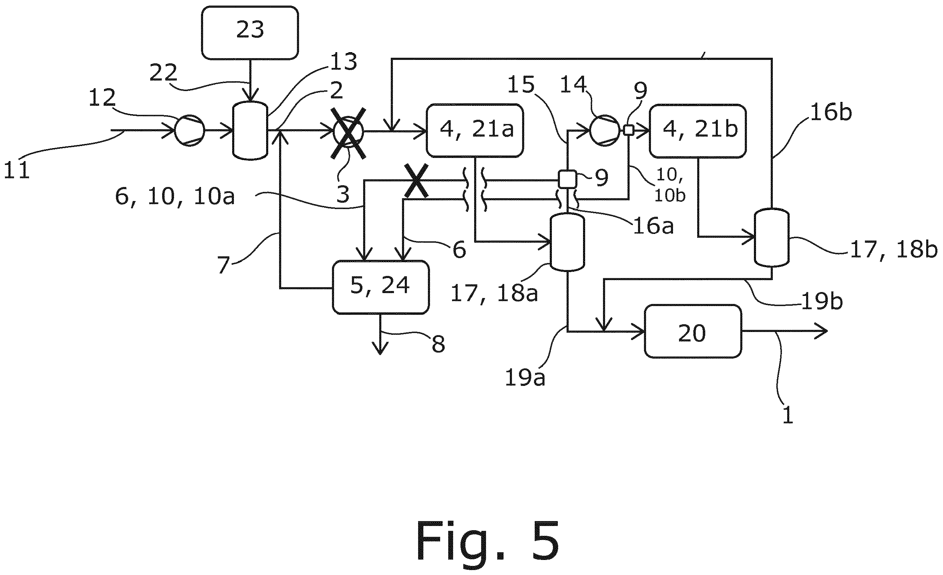

Das fünfte Ausführungsbeispiel der vorschlagsgemäßen Anlage der Fig. 5 verhält sich zu dem zweiten Ausführungsbeispiel der Fig. 2 sinngemäß gleich wie das vierte Ausführungsbeispiel der Fig. 4 zu dem ersten Ausführungsbeispiel der Fig. 1. Speziell ist der Zustand mit Betrieb des Synthesegaskompressors 3 der Anlage der Fig. 5 identisch zu dem Zustand mit Betrieb des Synthesegaskompressors 3 der Anlage der Fig. 2. Auf den Ausfall des Synthesegaskompressors 3 wird anders als bei der Anlage der Fig. 2 nicht die Führung des H-Recyclestroms 7 durch Umschalten verändert, sondern die des Rückgewinnungsstroms 6. Nach dem Umschalten der Leitungsanordnung 10 wird der Rückgewinnungsstrom 6 nicht mehr aus dem unreagierten Restgas 16a der ersten Reaktorstufe 21a vor der Druckerhöhung abgezweigt, sondern vielmehr aus dem von dem Recyclekompressor 14 druckerhöhten Restgasstrom 15. Die veränderte Führung entspricht wieder der ersten Teilleitung 10a der Leitungsanordnung 10 vor dem Umschalten und der zweiten Teilleitung 10b der Leitungsanordnung nach dem Umschalten.

Da der Recyclekompressor 14 hier prozesstechnisch zwischen der ersten Reaktorstufe 21a und der ihr nachgelagerten Reaktorstufe 21b angeordnet ist, wird also der Rückgewinnungsstrom 6 prozesstechnisch nach Druckerhöhung durch den Recyclekompressor 14 und vor Zufuhr zu der der ersten Reaktorstufe 21a nachgelagerten Reaktorstufe 21b abgezweigt. Wie bei dem Ausführungsbeispiel der Fig. 4 ergibt sich dadurch eine Druckerhöhung des der Wasserstoffrückgewinnungsanordnung 5 zugeführten Rückgewinnungsstroms 6.

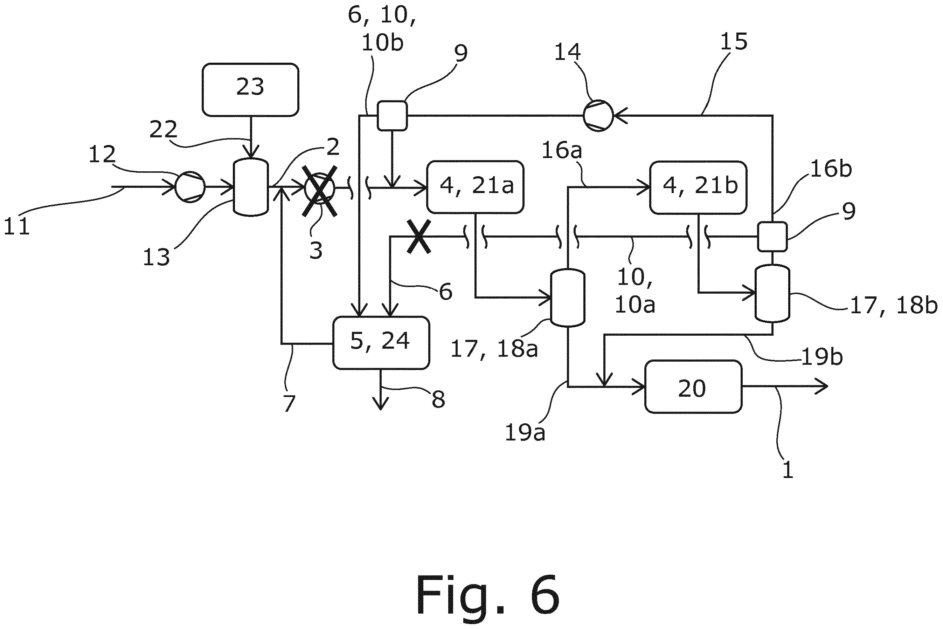

Das sechste Ausführungsbeispiel der vorschlagsgemäßen Anlage der Fig. 6 wiederum verhält sich zu dem dritten Ausführungsbeispiel der Fig. 3 sinngemäß gleich wie das vierte Ausführungsbeispiel der Fig. 4 zu demjenigen der Fig. 1 und das fünfte Ausführungsbeispiel der Fig. 5 zu demjenigen der Fig. 2. Das bedeutet, dass der Zustand mit Betrieb des Synthesegaskompressors 3 der Anlage der Fig. 6 identisch zu dem Zustand mit Betrieb des Synthesegaskompressors 3 der Anlage der Fig. 3 ist.

Auf den Ausfall des Synthesegaskompressors 3 wird anders als bei der Anlage der Fig. 3 nicht die Führung des H-Recyclestroms 7 durch Umschalten verändert, sondern die des Rückgewinnungsstroms 6. Nach dem Umschalten der Leitungsanordnung 10 wird der Rückgewinnungsstrom 6 nicht mehr aus dem unreagierten Restgas 16b der der ersten Reaktorstufe 21a nachgelagerten Reaktorstufe 21b abgezweigt, sondern vielmehr aus dem von dem Recyclekompressor 14 druckerhöhten Restgasstrom 15. Da die Anordnung des Recyclekompressors 14 derjenigen des Ausführungsbeispiels der Fig. 4 entspricht, wird also auch hier der Rückgewinnungsstrom 6 prozesstechnisch nach Druckerhöhung durch den Recyclekompressor 14 und vor Zufuhr zu der ersten Reaktorstufe 21a abgezweigt. Ebenso wie bei dem Ausführungsbeispiel der Fig. 4 ergibt sich dadurch eine Druckerhöhung des der Wasserstoffrückgewinnungsanordnung 5 zugeführten Rückgewinnungsstroms 6.

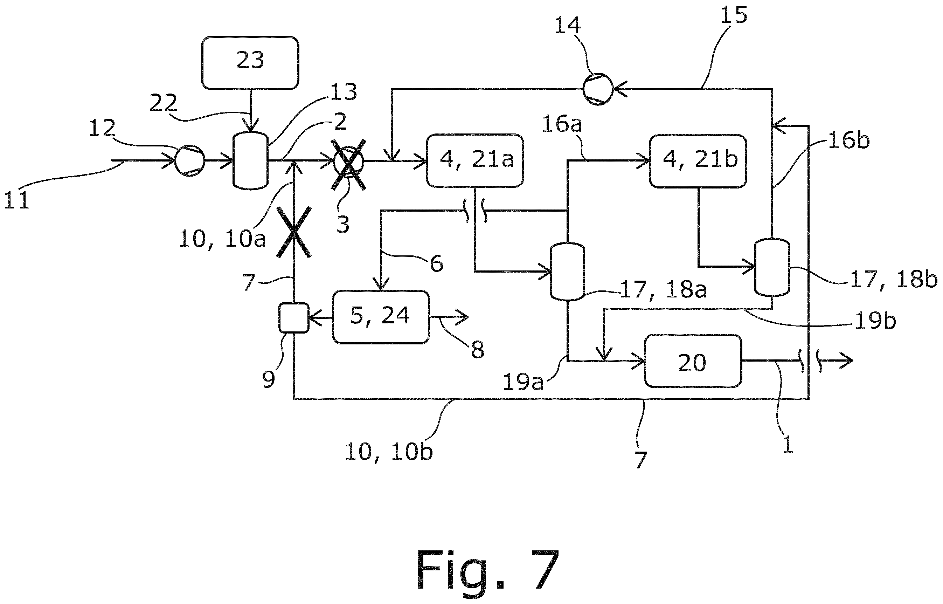

Schließlich entspricht das siebte Ausführungsbeispiel der vorschlagsgemäßen Anlage der Fig. 7 wieder dem ersten Ausführungsbeispiel der Fig. 1 in dem Zustand mit Synthesegaskompressor 3 im Betrieb. Wie bei dem Ausführungsbeispiel der Fig. 1 wird auch bei dem Ausführungsbeispiel der Fig. 7 auf einen Ausfall des Synthesegaskompressors 3 durch das Umschalten die Führung des H-Recyclestroms 7 verändert. Und zwar wird der H-Recyclestrom 7 nach dem Umschalten dem Restgasstrom 15, welcher hier aus dem Restgas 16b der der ersten Reaktorstufe 21a nachgelagerten Reaktorstufe 21b gewonnen wird, zugeführt. Speziell erfolgt diese Zuführung vor der Druckerhöhung durch den Recyclekompressor 14. Der Wasserstoff in dem H-Recyclestrom 7 erhält auf diese Weise eine Druckerhöhung durch den Recyclekompressor 14, was die durch den Ausfall des Synthesegaskompressors 3 fehlende Druckerhöhung zumindest teilweise kompensiert.

## Patentansprüche

1. Verfahren zum Betrieb einer Anlage für die Synthese von Methanol (1), wobei ein Synthesegasstrom (2) mit Wasserstoff und Kohlenstoffoxiden einem Synthesegaskompressor (3) der Anlage zur Druckerhöhung des Synthesegasstroms (2) zugeführt wird, wobei der druckerhöhte Synthesegasstrom (2) einer Methanol-Reaktoranordnung (4) der Anlage zur teilweisen Umwandlung in Methanol (1) zugeführt wird und wobei die Anlage eine Wasserstoffrückgewinnungsanordnung (5) aufweist, welche aus einem von der Methanol-Reaktoranordnung (4) zugeführten Rückgewinnungsstrom (6) einen H-Recyclestrom (7) mit Wasserstoff gewinnt, welcher Wasserstoff zumindest teilweise in Methanol umgewandelt wird, **dadurch gekennzeichnet, dass** auf einen Ausfall des Synthesegaskompressors (3) der Synthesegasstrom (2) weiterhin der Methanol-Reaktoranordnung (4) zur teilweisen Umwandlung in Methanol (1) zugeführt wird und dass nach dem Ausfall des Synthesegaskompressors (3) eine Leitungsanordnung (10) der Anlage derart umgeschaltet ist, dass der H-Recyclestrom (7) zur Kompensierung eines Druckverlusts in der Methanol-Reaktoranordnung (4) angepasst wird.

2. Verfahren nach Anspruch 1, **dadurch gekennzeichnet, dass** aus der Methanol-Reaktoranordnung (4) ein Restgasstrom (15) mit unreagiertem Restgas (16a, b) gewonnen wird und dass die Anlage einen Recyclekompressor (14) zur Druckerhöhung des Restgasstroms (15) und zur Zuführung des druckerhöhten Restgasstroms (15) zur Methanol-Reaktoranordnung (4) zur teilweisen Umwandlung in Methanol (1) aufweist, vorzugsweise, dass die Methanol-Reaktoranordnung (4) eine Methanol-Trennvorrichtung (17) zum Gewinnen des unreagierten Restgases (16a, b) und eines Rohmethanolstroms (19a, b) umfasst, insbesondere, dass die Methanol-Trennvorrichtung (17) eine Kondensationsvorrichtung (18a, b) zum Gewinnen des unreagierten Restgases (16a, b) und des Rohmethanolstroms (19a, b) durch Kondensation umfasst.

3. Verfahren nach Anspruch 2, **dadurch gekennzeichnet, dass** die Methanol-Reaktoranordnung (4) eine Vielzahl von prozesstechnisch hintereinander geschalteten Reaktorstufen (21a, b) zur Methanolsynthese aufweist, vorzugsweise, dass der Recyclekompressor (14) prozesstechnisch zwischen zwei Reaktorstufen (21a, b) angeordnet ist.

4. Verfahren nach Anspruch 3, **dadurch gekennzeichnet, dass** der druckerhöhte Synthesegasstrom (2) einer ersten Reaktorstufe (21a) der Vielzahl von Reaktorstufen (21a, b) zugeführt wird, vorzugsweise, dass der Restgasstrom (15) aus einer prozesstechnisch der ersten Reaktorstufe (21a) nachgelagerten Reaktorstufe (21b) gewonnen wird, insbesondere, dass der Recyclekompressor (14) den druckerhöhten Restgasstrom (15) der ersten Reaktorstufe (21a) zuführt.

5. Verfahren nach einem der Ansprüche 2 bis 4, **dadurch gekennzeichnet, dass** nach dem Umschalten der Leitungsanordnung (10) der H-Recyclestrom (7) dem unreagierten Restgas (16a, b) zugeführt wird, vorzugsweise, dass nach dem Umschalten der Leitungsanordnung (10) der H-Recyclestrom (7) dem Recyclekompressor (14) zur Druckerhöhung gemeinsam mit dem Restgasstrom (15) zugeführt wird.

6. Verfahren nach einem der Ansprüche 1 bis 5, **dadurch gekennzeichnet, dass** durch das Umschalten der Leitungsanordnung (10) der Verlauf des Rückgewinnungsstroms (6) geändert wird, vorzugsweise, dass vor dem Umschalten der Leitungsanordnung (10) der Rückgewinnungsstrom (6) aus dem unreagierten Restgas (16a, b), insbesondere dem Recyclekompressor (14) prozesstechnisch vorgelagert, abgezweigt wird.

7. Verfahren nach einem der Ansprüche 1 bis 6, dass durch das Umschalten der Leitungsanordnung (10) der Rückgewinnungsstrom (6) mit einem erhöhten Druck der Wasserstoffrückgewinnungsanordnung (5) zugeführt wird, vorzugsweise, dass durch das Umschalten der Leitungsanordnung (10) der Rückgewinnungsstrom (6) mit einem höheren Druck als vor dem Umschalten der Leitungsanordnung (10) zugeführt wird, insbesondere, dass nach dem Umschalten der Leitungsanordnung (10) der Rückgewinnungsstrom (6) dem Recyclekompressor (14) prozesstechnisch nachgelagert aus dem Restgasstrom (16a, b) abgezweigt wird.

8. Verfahren nach einem der Ansprüche 1 bis 7, **dadurch gekennzeichnet, dass** durch das Umschalten der Leitungsanordnung (10) der Verlauf des H-Recyclestroms (7) geändert wird, vorzugsweise, dass vor dem Umschalten der Leitungsanordnung (10) der H-Recyclestrom (7) dem Synthesegasstrom (2) dem Synthesegaskompressor (3) prozesstechnisch vorgelagert zugeführt wird.

9. Verfahren nach einem der Ansprüche 1 bis 8, **dadurch gekennzeichnet, dass** durch das Umschalten der Leitungsanordnung (10) der Wasserstoff des H-Recyclestroms (7) der Methanol-Reaktoranordnung (4) mit einem erhöhten Druck zur teilweisen Umwandlung in Methanol (1) zugeführt wird, vorzugsweise, dass durch das Umschalten der Leitungsanordnung (10) der Wasserstoff des H-Recyclestroms (7), vorzugsweise der H-Recyclestrom (7), der Methanol-Reaktoranordnung (4) mit einem höheren Druck als, insbesondere nach Ausfall des Synthesegaskompressors (3), vor dem Umschalten der Leitungsanordnung (10) zugeführt wird.

10. Verfahren nach einem der Ansprüche 1 bis 9, **dadurch gekennzeichnet, dass** der Synthesegasstrom (2) in einer Synthesegasreaktoranordnung (13) der Anlage aus einem kohlenstoffhaltigen Energieträgerstrom (11) gewonnen wird, vorzugsweise, dass zum Gewinnen des Synthesegasstroms (2) ein sauerstoffhaltiger Strom (22) der Synthesegasreaktoranordnung (13) zugeführt wird, insbesondere, dass vor dem Umschalten der H-Recyclestrom (7) dem Synthesegasstrom (2) der Synthesegasreaktoranordnung (13) prozesstechnisch nachgelagert zugeführt wird.

11. Verfahren nach Anspruch 10, **dadurch gekennzeichnet, dass** in der Synthesegasreaktoranordnung (13) der Synthesegasstrom (2) durch eine autotherme Reformierung aus dem kohlenstoffhaltigen Energieträgerstrom (11) gewonnen wird, vorzugsweise, dass der sauerstoffhaltige Strom (22) aus einer Luftzerlegungsvorrichtung (23) zum Gewinnen eines Sauerstoffstroms aus einer Umgebungsluft gewonnen wird, insbesondere, dass der sauerstoffhaltige Strom (22) im Wesentlichen aus Sauerstoff besteht.

12. Verfahren nach Anspruch 10 oder 11, **dadurch gekennzeichnet, dass** nach dem Umschalten der Leitungsanordnung der H-Recyclestrom (7) dem Energieträgerstrom (11), insbesondere der Synthesegasreaktoranordnung (13) prozesstechnisch vorgelagert, zugeführt wird, weiter insbesondere, dass die Anlage einen Energieträgerkompressor (12) zur Druckerhöhung des Energieträgerstroms (11) vor Zuführung zu der Synthesegasreaktoranordnung (13) aufweist und dass nach dem Umschalten der Leitungsanordnung (10) der H-Recyclestrom (7) dem Energieträgerstrom (11) dem Energieträgerkompressor (12) vorgelagert zugeführt wird, weiter vorzugsweise, dass die Wasserstoffrückgewinnungsanordnung (5) einen Purgestrom (8) ausgibt, welcher weiter insbesondere zur Verfeuerung abgeführt wird.

13. Verfahren nach einem der Ansprüche 1 bis 12, **dadurch gekennzeichnet, dass** der H-Recyclestrom (7) einen höheren molaren Anteil an Wasserstoff als der Rückgewinnungsstrom (6) aufweist, vorzugsweise, dass der H-Recyclestrom (7) einen höheren molaren Anteil an Wasserstoff als der Purgestrom (8) aufweist.

14. Verfahren nach einem der Ansprüche 1 bis 13, **dadurch gekennzeichnet, dass** die Wasserstoffrückgewinnungsanordnung (5) eine Druckwechsel-Adsorptionsvorrichtung (24) zum Gewinnen des H-Recyclestroms (7) aus dem Rückgewinnungsstrom (6) aufweist, weiter vorzugsweise, dass der H-Recyclestrom (7) im Wesentlichen aus Wasserstoff besteht.

15. Anlage für die Synthese von Methanol (1) mit einem Synthesegaskompressor (3), welchem ein Synthesegasstrom (2) mit Wasserstoff und Kohlenstoffoxiden zur Druckerhöhung des Synthesegasstroms (2) zugeführt wird, mit einer Methanol-Reaktoranordnung (4), welcher der druckerhöhte Synthesegasstrom (2) zur teilweisen Umwandlung in Methanol (1) zugeführt wird und mit einer Wasserstoffrückgewinnungsanordnung (5) zum Gewinnen eines H-Recyclestroms (7) mit Wasserstoff aus einem von der Methanol-Reaktoranordnung (4) zugeführten Rückgewinnungsstrom (6), wobei der Wasserstoff zumindest teilweise in Methanol umgewandelt wird, **dadurch gekennzeichnet, dass** die Anlage eine Leitungsanordnung (10) und eine Schaltanordnung (9) aufweist, welche auf einen Ausfall des Synthesegaskompressors (3) die Leitungsanordnung (10) derart umschaltet, dass der H-Recyclestrom (7) zur Kompensierung eines Druckverlusts in der Methanol-Reaktoranordnung (4) angepasst wird.

## Claims

1. A method for operating a system for the synthesis of methanol (1), wherein a stream of synthesis gas (2) containing hydrogen and oxides of carbon is supplied to a synthesis gas compressor (3) of the system in order to raise the pressure of the synthesis gas stream (2), wherein the pressurised synthesis gas stream (2) is supplied to a methanol reactor assembly (4) of the system for partial conversion into methanol (1), and wherein the system has a hydrogen recovery assembly (5) which produces a H recycle stream (7) containing hydrogen from a recovery stream (6) supplied from the methanol reactor assembly (4), at least a portion of which hydrogen being converted into methanol, **characterized in that** in the event of a breakdown of the synthesis gas compressor (3), the synthesis gas stream (2) continues to be supplied to the methanol reactor assembly (4) for partial conversion into methanol (1), and **in that** after the breakdown of the synthesis gas compressor (3), a pipeline assembly (10) of the system is switched in, in a manner such that the H recycle stream (7) is adjusted in order to compensate for a loss of pressure in the methanol reactor assembly (4).

2. The method as claimed in claim 1, **characterized in that** a residual gas stream (15) containing unreacted residual gas (16a, b) is obtained from the methanol reactor assembly (4) and **in that** the system has a recycle compressor (14) in order to raise the pressure of the residual gas stream (15) and in order to supply the pressurised residual gas stream (15) to the methanol reactor assembly (4) for partial conversion into methanol (1), preferably **in that** the methanol reactor assembly (4) comprises a methanol separation device (17) in order to obtain the unreacted residual gas (16a, b) and an unrefined methanol stream (19a, b), in particular **in that** the methanol separation device (17) comprises a condensation device (18a, b) in order to obtain the unreacted residual gas (16a, b) and the unrefined methanol stream (19a, b) by means of condensation.

3. The method as claimed in claim 2, **characterized in that** the methanol reactor assembly (4) has a plurality of reactor stages (21a, b) for the synthesis of methanol which are procedurally connected one behind the other, preferably **in that** the recycle compressor (14) is procedurally disposed between two reactor stages (21a, b).

4. The method as claimed in claim 3, **characterized in that** the pressurised synthesis gas stream (2) is supplied to a first reactor stage (21a) of the plurality of reactor stages (21a, b), preferably **in that** the residual gas stream (15) is obtained from a reactor stage (21b) which is procedurally downstream of the first reactor stage (21a), in particular **in that** the recycle compressor (14) supplies the pressurised residual gas stream (15) to the first reactor stage (21a).

5. The method as claimed in one of claims 2 to 4, **characterized in that** after switching in the pipeline assembly (10), the H recycle stream (7) is supplied to the unreacted residual gas (16a, b), preferably **in that** after switching in the pipeline assembly (10), the H recycle stream (7) is supplied to the recycle compressor (14) in order to raise the pressure together with the residual gas stream (15).

6. The method as claimed in one of claims 1 to 5, **characterized in that** by switching in the pipeline assembly (10), the path of the recovery stream (6) is modified, preferably **in that** prior to switching in the pipeline assembly (10), the recovery stream (6) is branched off from the unreacted residual gas (16a, b), in particular procedurally upstream of the recycle compressor (14).

7. The method as claimed in one of claims 1 to 6, **characterized in that** by switching in the pipeline assembly (10), the recovery stream (6) is supplied at an increased pressure to the hydrogen recovery assembly (5), preferably **in that** by switching in the pipeline assembly (10), the recovery stream (6) is supplied at a higher pressure than prior to switching in the pipeline assembly (10), in particular **in that** after switching in the pipeline assembly (10), the recovery stream (6) is branched off from the residual gas stream (16a, b) procedurally downstream of the recycle compressor (14).

8. The method as claimed in one of claims 1 to 7, **characterized in that** by switching in the pipeline assembly (10), the path of the H recycle stream (7) is modified, preferably **in that** prior to switching in the pipeline assembly (10), the H recycle stream (7) is supplied to the synthesis gas stream (2) procedurally upstream of the synthesis gas compressor (3).

9. The method as claimed in one of claims 1 to 8, **characterized in that** by switching in the pipeline assembly (10), the hydrogen from the H recycle stream (7) is supplied to the methanol reactor assembly (4) at an increased pressure for partial conversion into methanol (1), preferably **in that** by switching in the pipeline assembly (10), the hydrogen from the H recycle stream (7), preferably the H recycle stream (7), is supplied to the methanol reactor assembly (4) at a higher pressure than prior to switching in the pipeline assembly (10), in particular after breakdown of the synthesis gas compressor (3).

10. The method as claimed in one of claims 1 to 9, **characterized in that** the synthesis gas stream (2) is produced in a synthesis gas reactor assembly (13) of the system from a carbon-containing energy source stream (11), preferably **in that**, in order to produce the synthesis gas stream (2), an oxygen-containing stream (22) is supplied to the synthesis gas reactor assembly (13), in particular **in that** prior to switching in, the H recycle stream (7) is supplied to the synthesis gas stream (2) procedurally downstream of the synthesis gas reactor assembly (13) .

11. The method as claimed in claim 10, **characterized in that** in the synthesis gas reactor assembly (13), the synthesis gas stream (2) is produced by autothermal reforming of the carbon-containing energy source stream (11), preferably **in that** the oxygen-containing stream (22) is produced from an air separation device (23) for producing a stream of oxygen from environmental air, in particular **in that** the oxygen-containing stream (22) substantially consists of oxygen.

12. The method as claimed in claim 10 or claim 11, **characterized in that** after switching in the pipeline assembly, the recycle stream (7) is supplied to the energy source stream (11), in particular procedurally upstream of the synthesis gas reactor assembly (13), more particularly **in that** the system has an energy source compressor (12) in order to increase the pressure of the energy source stream (11) prior to supply to the synthesis gas reactor assembly (13), and **in that** after switching in the pipeline assembly (10), the H recycle stream (7) is supplied to the energy source stream (11) upstream of the energy source compressor (12), more preferably **in that** the hydrogen recovery assembly (5) outputs a purge stream (8) which, more particularly, is discharged for combustion.

13. The method as claimed in one of claims 1 to 12, **characterized in that** the H recycle stream (7) has a higher molar proportion of hydrogen than the recovery stream (6), preferably **in that** the H recycle stream (7) has a higher molar proportion of hydrogen than the purge stream (8).

14. The method as claimed in one of claims 1 to 13, **characterized in that** the hydrogen recovery assembly (5) has a pressure swing adsorption device (24) for producing the H recycle stream (7) from the recovery stream (6), more preferably **in that** the H recycle stream (7) substantially consists of hydrogen.

15. A system for the synthesis of methanol (1), with a synthesis gas compressor (3) to which a synthesis gas stream (2) containing hydrogen and oxides of carbon is supplied in order to increase the pressure of the synthesis gas stream (2), with a methanol reactor assembly (4) to which the pressurised synthesis gas stream (2) is supplied for partial conversion into methanol (1), and with a hydrogen recovery assembly (5) for the production of a H recycle stream (7) containing hydrogen from a recycle stream (6) supplied from the methanol reactor assembly (4), wherein at least a portion of the hydrogen is converted into methanol, **characterized in that** the system has a pipeline assembly (10) and a switching assembly (9) which switches in the pipeline assembly (10) in the event of a breakdown of the synthesis gas compressor (3) in a manner such that the H recycle stream (7) is adjusted in order to compensate for a loss of pressure in the methanol reactor assembly (4).

## Revendications

1. Procédé opérationnel d'une installation de synthèse du méthanol (1), un flux de gaz de synthèse (2) étant amené avec de l'hydrogène et des oxydes de carbone vers un compresseur de gaz de synthèse (3) de l'installation pour élever la pression du flux de gaz de synthèse (2), le flux de gaz de synthèse (2) élevé en pression étant amené vers un ensemble de réacteurs à méthanol (4) de l'installation pour sa transformation partielle en méthanol (1) et l'installation comportant un ensemble de recyclage d'hydrogène (5) qui récupère à partir d'un flux recyclé (6) ramené à partir de l'ensemble de réacteurs à méthanol (4) un flux de H recyclé (7) avec de l'hydrogène, lequel hydrogène est transformé au moins partiellement en méthanol, **caractérisé en ce que** suite à une défaillance du compresseur de gaz de synthèse (3), le flux de gaz de synthèse (2) continue à être amené vers l'ensemble de réacteurs à méthanol (4) pour sa transformation partielle en méthanol (1) et **en ce qu'**après la défaillance du compresseur de gaz de synthèse (3), un ensemble de conduites (10) de l'installation est commuté de telle sorte que le flux de H recyclé (7) soit adapté, pour compenser une perte de pression dans l'ensemble de réacteurs à méthanol (4) .

2. Procédé selon la revendication 1, **caractérisé en ce que** l'on récupère à partir de l'ensemble de réacteurs à méthanol (4) un flux de gaz résiduel (15) de gaz résiduel (16a, b) n'ayant pas réagi et **en ce que** l'installation comporte un compresseur de recyclage (14), destiné à élever la pression du flux de gaz résiduel (15) et à amener le flux de gaz résiduel (15) vers l'ensemble de réacteurs à méthanol (4) pour sa transformation partielle en méthanol (1), de préférence **en ce que** l'ensemble de réacteurs à méthanol (4) comprend un dispositif séparateur de méthanol (17), destiné à récupérer le gaz résiduel (16a, b) n'ayant pas réagi et un flux de méthanol brut (19a, b), notamment **en ce que** le dispositif séparateur de méthanol (17) comprend un dispositif de condensation (18a, b), destiné à récupérer par condensation le gaz résiduel (16a, b) n'ayant pas réagi et le flux de méthanol brut (19a, b).

3. Procédé selon la revendication 2, **caractérisé en ce que** l'ensemble de réacteurs à méthanol (4) comporte une pluralité d'étages de réacteur (21a, b) montés les uns derrière les autres selon la technique du processus pour la synthèse du méthanol, de préférence **en ce que** le compresseur de recyclage (14) est placé selon la technique du processus entre deux étages de réacteur (21a, b).

4. Procédé selon la revendication 3, **caractérisé en ce que** l'on amène le flux de gaz de synthèse (2) élevé en pression vers un premier étage de réacteur (21a) parmi la pluralité d'étages de réacteur (21a, b), de préférence **en ce que** l'on récupère le flux de gaz résiduel (15) à partir d'un étage de réacteur (21b) monté en aval du premier étage de réacteur (21a) selon la technique du processus, notamment **en ce que** le compresseur de recyclage (14) amène le flux de gaz résiduel (15) élevé en pression vers le premier étage de réacteur (21a).

5. Procédé selon l'une quelconque des revendications 2 à 4, **caractérisé en ce qu'**après la commutation de l'ensemble de conduites (10), l'on amène le flux de H recyclé (7) vers le gaz résiduel (16a, b) n'ayant pas réagi, de préférence **en ce qu'**après la commutation de l'ensemble de conduites (10), l'on amène le flux de H recyclé (7) vers le compresseur de recyclage (14) pour en élever la pression, conjointement avec le flux de gaz résiduel (15).

6. Procédé selon l'une quelconque des revendications 1 à 5, **caractérisé en ce que** la commutation de l'ensemble de conduites (10) a pour effet de modifier le trajet du flux recyclé (6), de préférence **en ce qu'**avant la commutation de l'ensemble de conduites (10), le flux recyclé (6) est dérivé du gaz résiduel (16a, b) n'ayant pas réagi, notamment selon la technique du processus en amont du compresseur de recyclage (14).

7. Procédé selon l'une quelconque des revendications 1 à 6, **caractérisé en ce que** suite à la commutation de l'ensemble de conduites (10), le flux recyclé (6) est amené avec une pression élevée vers l'ensemble de recyclage d'hydrogène (5), de préférence, **en ce que** suite à la commutation de l'ensemble de conduites, le flux recyclé (6) est amené vers l'ensemble de conduites (10) avec une pression plus élevée qu'avant la commutation, notamment **en ce qu'**après la commutation de l'ensemble de conduites (10), le flux recyclé (6) est dérivé du flux de gaz résiduel (16a, b), en aval du compresseur de recyclage (14) selon la technique du processus.

8. Procédé selon l'une quelconque des revendications 1 à 7, **caractérisé en ce que** suite à la commutation de l'ensemble de conduites (10), le trajet du flux de H recyclé (7) est modifié, de préférence **en ce qu'**avant la commutation de l'ensemble de conduites (10), l'on amène le flux de H recyclé (7) vers le flux de gaz de synthèse (2) en amont du compresseur de gaz de synthèse (3) selon la technique du processus.

9. Procédé selon l'une quelconque des revendications 1 à 8, **caractérisé en ce que** suite à la commutation de l'ensemble de conduites (10) l'on amène l'hydrogène du flux de H recyclé (7) avec une pression élevée vers l'ensemble de réacteurs à méthanol (4) pour sa transformation partielle en méthanol (1), de préférence **en ce que** suite à la commutation de l'ensemble de conduites (10) l'on amène l'hydrogène du flux de H recyclé (7), de préférence le flux de H recyclé (7) vers l'ensemble de réacteurs à méthanol (4) avec une pression plus élevée notamment après la défaillance du compresseur de gaz de synthèse (3) qu'avant la commutation de l'ensemble de conduites (10) .

10. Procédé selon l'une quelconque des revendications 1 à 9, **caractérisé en ce que** l'on récupère le flux de gaz de synthèse (2) dans un ensemble de réacteurs de gaz de synthèse (13) de l'installation à partir d'un flux de sources d'énergie (11) carboné, de préférence **en ce que** pour récupérer le flux de gaz de synthèse (2), l'on amène vers l'ensemble de réacteurs de gaz de synthèse (13) un flux (22) oxygéné, notamment **en ce qu'**avant la commutation, l'on amène le flux de H recyclé (7) vers le flux de gaz de synthèse (2), en aval de l'ensemble de réacteurs de gaz de synthèse (13) selon la technique du processus.

11. Procédé selon la revendication 10, **caractérisé en ce que** dans l'ensemble de réacteurs de gaz de synthèse (13), l'on récupère le flux de gaz de synthèse (2) par reformage autothermique à partir du flux de sources d'énergie (11) carboné, de préférence **en ce que** l'on récupère le flux (22) oxygéné à partir d'un dispositif séparateur d'air (23), destiné à récupérer un flux d'oxygène à partir d'un air ambiant, notamment **en ce que** le flux (22) oxygéné est majoritairement constitué d'oxygène.

12. Procédé selon la revendication 10 ou 11, **caractérisé en ce qu'**après la commutation de l'ensemble de conduites, l'on amène le flux de H recyclé (7) vers le flux de sources d'énergie (11), notamment en amont de l'ensemble de réacteurs de gaz de synthèse (13) selon la technique du processus, notamment par ailleurs **en ce que** l'installation comporte un compresseur de sources d'énergie (12), destiné à élever la pression du flux de sources d'énergie (11) avant l'amenage vers l'ensemble de réacteurs de gaz de synthèse (13) et **en ce qu'**après la commutation de l'ensemble de conduites (10), l'on amène le flux de H recyclé (7) vers le flux de sources d'énergie (11), en amont du compresseur de sources d'énergie (12), de préférence par ailleurs, **en ce que** l'ensemble de recyclage d'hydrogène (5) délivre un flux de purge (8) lequel est évacué notamment par ailleurs pour être brûlé.

13. Procédé selon l'une quelconque des revendications 1 à 12, **caractérisé en ce que** le flux de H recyclé (7) comporte une part moléculaire plus élevée d'hydrogène que le flux recyclé (6), de préférence **en ce que** le flux de H recyclé (7) comporte une part moléculaire plus élevée d'hydrogène que le flux de purge (8).

14. Procédé selon l'une quelconque des revendications 1 à 13, **caractérisé en ce que** l'ensemble de recyclage d'hydrogène (5) comporte un dispositif d'adsorption par changement de pression (24), destiné à récupérer le flux de H recyclé (7) à partir du flux recyclé (6), de préférence par ailleurs, **en ce que** le flux de H recyclé (7) est majoritairement constitué d'hydrogène.

15. Installation destinée à la synthèse méthanol (1), dotée d'un compresseur de gaz de synthèse (3) auquel est amené un flux de gaz de synthèse (2) avec de l'hydrogène et des oxydes de carbone pour élever la pression du flux de gaz de synthèse (2), dotée d'un ensemble de réacteurs à méthanol (4), vers lequel le flux de gaz de synthèse (2) élevé en pression est amené pour sa transformation partielle en méthanol (1), dotée d'un ensemble de recyclage d'hydrogène (5), destiné à récupérer un flux de H recyclé (7) avec de l'hydrogène à partir d'un flux recyclé (6) amené par l'ensemble de réacteurs à méthanol (4), l'hydrogène étant transformé au moins partiellement en méthanol, **caractérisé en ce que** l'installation comporte un ensemble de conduites (10) et un ensemble de commutation (9), qui suite à une défaillance du compresseur de gaz de synthèse (3) commute l'ensemble de conduites (10) de telle sorte que le flux de H recyclé (7) soit adapté pour compenser une perte de pression dans l'ensemble de réacteurs à méthanol (4).
